# EUROPÄISCHE PATENTSCHRIFT

(11) **EP 1 330 291 B1**
(45) Veröffentlichungstag und Bekanntmachung des Hinweises auf die Patenterteilung: **02.02.2005**
(21) Anmeldenummer: 01992597.3
(22) Anmeldetag: 05.11.2001
(51) Int. Cl.: A61N 5/067

(54) **VORRICHTUNG ZUR REINIGUNG VON WUNDEN MITTELS LASER**
DEVICE FOR THE CLEANING OF WOUNDS BY MEANS OF LASER
DISPOSITIF POUR LE NETTOYAGE DE PLAIES AU LASER

(30) Priorität: 05.11.2000 DE 10055677
(43) Veröffentlichungstag der Anmeldung: 30.07.2003
(73) Patentinhaber: Clyxon Laser GmbH, 12489 Berlin (DE)
(72) Erfinder: SCHÖNBORN, Karl-Heinz, 12355 Berlin (DE)
(74) Vertreter: Gross, Felix, Dr.
(86) Internationale Anmeldenummer: PCT/DE2001/004179
(87) Internationale Veröffentlichungsnummer: WO 2002/036200

(56) Entgegenhaltungen:
- WO-A-94/23478
- WO-A-98/57588
- WO-A-99/16369
- US-A- 5 021 452
- US-A- 5 259 380
- US-A- 5 445 146
- US-A- 5 662 644
- US-A- 5 951 596
- KUPER J.W. ET AL: 'MEDICAL APPLICATIONS OF ALEXANDRITE LASER SYSTEMS' TOPICAL MEETING ON TUNABLE SOLID STATE LASERS - TECHNICAL DIGEST 1987, Seiten 183 - 184

## Beschreibung

### Aufgabe der Erfindung

Das Reinigen von Verletzungswunden von menschlichen oder tierischen Unfallopfern (Straßenverkehr, Industrie und Haushalt) stellt immer noch ein großes Problem dar.

Häufig werden bei offenen Wunden Fremdkörper und Verunreinigungen in die offenen Wunden hereingetragen, was zu gefährlichen Entzündungen führen kann. Teilweise werden auch Fremdkörper in die Hautoberfläche "hineintätowiert".

Auch nach einer Reinigung der Wunde besteht die Gefahr, dass Keime in den Wundbereich einwandern und zur Entzündung führen. Ziel muss es daher sein, Fremdkörper aus der Wunde zu entfernen, die Wunde und die angrenzenden Hautareale zu desinfizieren und diesen Zustand durch einen geeigneten Verband zu konservieren.

Diese Vorbereitung für die Versorgung der Wunde stellt die beste Voraussetzung dar, um eine schnelle und komplikationslose Heilung zu fördern.

Bisher wird die Reinigung von Wunden durch Spülungen und mechanische Reinigungsprozesse wie z. B. Wischen oder auch Bürsten, durchgeführt. Diese groben Methoden sind aufwendig, fügen der Wunde zusätzliche Schädigungen hinzu und liefern nicht die geforderte Keimfreiheit von Wunde und Wundumgebung.

Aus der Patentschrift US-A-5 021 452 ist es bekannt, Laserstrahlung mit niedriger Leistung im mit einer Wellenlänge im Bereich von 600 bis 1100 nm zur Wundheilung einzusetzen.

Die Behandlung mit kurzgepulster Laserstrahlung hoher Leistungsdichte ist auch ein gutes Verfahren, um die Keimbelastung am Gewebe zu verringern, also eine weitgehende Keimfreiheit zu erzielen.

### Beschreibung der Erfindung

Zur Reinigung und Keimreduktion der Wunde wird die

Wundoberfläche eventuell auch die angrenzenden Hautareale mit gepulster Laserstrahlung eines Vorrichtung gemäß Anspruch 1 beaufschlagt.

Die Laserstrahlung wirkt gezielt auf die Verschmutzungen und Fremdkörper und entfernt diese durch Vaporisation aus der Wunde.

Durch die eine bestimmte Wahl der Wellenlänge und des zeitlichen Pulsverlaufes wird die Wunde selbst nur wenig beeinflusst. Insbesondere wird sie durch die Vorrichtung nur wenig, d. h. nur bis maximal ca. 50°C erwärmt, so dass einer offenen Schürf- oder Schnittwunde nicht durch thermische Effekte eine Brandverletzung hinzugefügt wird.

Die Laserstrahlung wird mit solchen Strahldaten gewählt, dass die Strahlung im Blut und im verletzten Gewebe nicht stark absorbiert wird, um diese nicht unnötig aufzuheizen.

Vorteilhaft ist es, wenn eine Laserabstrahlung mit kurzen Pulsen von unterhalb 100*µ*s gewählt wird, damit die Laserenergie durch selektive Absorption die Schmutzpartikel schlagartig verdampft und abträgt, ohne die Wunde selbst übermäßig zu erwärmen. Besonders vorteilhaft sind überraschend Pulsdauern von unterhalb 2,5µs.

Die gepulste Laserstrahlung wirkt durch Zell-Schädigung der unerwünschten Fremdzellen keimreduzierend, wenn sie auf Gewebe appliziert wird. Dies ist ein zusätzlicher gewünschter Effekt der Erfindung.

Als zusätzliche Maßnahme wird gegebenenfalls mit keimfreiem Spülgas, z. B. mit keimfreier Luft oder mit keimfreiem Inertgas, der Wirkbereich der Laserstrahlung beströmt, um die Zersetzungsprodukte abzutransportieren und den Wundbereich zu kühlen.

Alternativ kann auch mit einem vernebelten Strahl von keimfreier Spülflüssigkeit, z. B. keimfreiem Wasser oder keimfreier physiologischer Salzlösung, gegebenenfalls unter Zugabe von antiseptischen Mitteln oder heilungsfördernden Medikamenten, die Wunde gekühlt werden.

Der Spülmittelstrahl kann alternativ synchron mit und ausgerichtet zum Laserstrahl auf die Wundoberfläche gerichtet werden, um.die Reinigungswirkung zu verstärken.

Die Kühlwirkung kann vorteilhafterweise verstärkt werden, indem Spülgas oder Spülflüssigkeit in vorgekühltem Zustand appliziert werden oder indem dem Spülmittel ein Kühlmittel beigefügt wird, das durch Verdampfung auf der Wundoberfläche seine Kühlwirkung entfaltet.

Die Applikation geschieht vorzugsweise über ein Handstück, das
1. über eine optische Faser oder einen Spiegelgelenkarm vom Laser mit Strahlung versorgt wird,
2. die Laserstrahlung kollimiert oder fokussiert auf die Zielfläche richtet und
3. die notwendigen Schlauchführungen und Düsen enthält, um die genannten Spül- und Kühlmedien auf den Wirkpunkt des Laserstrahls zu applizieren.

### Bevorzugt einzusetzende Lasertypen

Besonders wirksam und gleichzeitig kostengünstig ist ein gütegeschalteter Nd:YAG-Laser entweder im gepulsten Betrieb oder mit akusto-optischer Modulation bei cw-Betrieb des Lasers. Im letztgenannten Fall sind auch diodengepumpte Laser auf der Basis eines Neodym-dotierten Laserkristalls vorteilhaft einzusetzen.

Besonders vorteilhaft sind, wie sich überraschenderweise zeigt, Laser einer der genannten Bauarten, die bei der Laserlinie im Bereich 1300 bis 1550 nm betrieben werden, beim Nd:YAG speziell die Wellenlänge 1330 nm.

Eine Steigerung der Wirkung ist durch Frequenzverdoppelung zu erzielen, das ist beim Nd:YAG-Laser mit Grundwellenlänge 1330 nm die Wellenlänge 665 nm:

Die selektive Absorption in Schmutzpartikeln z. T. ist besser als bei 1330 nm, während die Absorption im Blut der offenen Wunde wunschgemäß gering ist.

Eine besonders günstige Anordnung der Erfindung ist die, dass bei Frequenzverdoppelung eines der genannten Neodym-dotierten Kristall-Laser (bevorzugt Nd:YAG) durch geeignete Auslegung des Auskoppelspiegels sowohl ein Teil der Energie bei 1330 nm als auch der zweiten Harmonischen bei 665 nm appliziert wird.

Die heterogenen Schmutzpartikel absorbieren überwiegend wenigstens bei einer der Wellenlängen, während die Absorption in Blut und im Gewebe für beide Wellenlängen moderat bleibt.

Die genannten Laser lassen sich bei nennenswerten Wirkenergien nur sehr begrenzt durch Quarzglasfasern übertragen, so dass nur eine direkte Applikation oder ein Spiegelgelenkarm möglich ist.

### Faserverlängerter Resonator

In EP 0 691 043 ist ein passiv gütegeschaltetes Lasersystem beschrieben, das als Besonderheit eine Intra-Cavity-Fiber aufweist. Diese optische Fiber ist in den Laserresonator integriert, so dass ein Resonator großer Länge (10-50m) und entsprechend großer Resonatorumlaufzeit entsteht.

Durch diesen Aufbau werden je nach Auslegung Pulsdauern von 200 ns bis über 1 *µ*s erzielt, die sonst weder von passiv gütegeschalteten, noch von freilaufenden Lasern erzielbar sind.

Bei Experimenten zum Einsatz eines solchen Lasers in der Medizin hat sich nun überraschend gezeigt, dass dieser Laseraufbau für die hier beschriebene Indikation besonders vorteilhaft ist.

Stellt man die Pulsdauer bei Pulsenergien von 50 bis 100 mJ auf Pulsdauern von 200 bis 600 ns ein, so erhält man für die Wundreinigung ausreichend kurze und vorteilhafte Pulsdauern, wobei gleichzeitig eine Übertragung durch Quarzglasfasern möglich ist.

Dies führt zu einem besonders flexibel und unkompliziert einsetzbaren Gerät.

Die Wellenlängen 1330 und 665 nm ergeben bei diesem Aufbau eine günstige Kombination, als ca. 15-30% der Energie in die zweite Harmonische transformiert und synchron mit dem Puls der Grundwellenlänge abgestrahlt werden.

### Strahldaten im Applikationsgebiet

Im Anwendungsfeld, d. h. auf der Oberfläche der Wunde oder der zu behandelnden verletzten Körperoberfläche werden durch die genannten Laser Energiedichten von 10J/mm² oder höher erzielt. Zusammen mit den kurzen Pulsdauern ergeben sich im Laserpuls Leistungsdichten von 100 kW/mm²oder mehr, bevorzugt von größer als 5 MW/mm².

Dabei bleibt bei Pulsrepetitionsraten von 1 bis 100 Hz die Dichte der mittleren Leistung moderat, so daß die thermischen Effekte limitiert bleiben.

Die Wirkung auf das Gewebe und die gezielte Abdampfung der Verschmutzung erfolgt durch die gewählten Daten primär durch photoablative Prozesse oder sonstige nichtlineare und mit hoher Laserleistungsdichte verbundene Wirkmechanismen.

Nicht auszuschließen ist selbstverständlich als positive Begleiterscheinung eine biostimulative Wirkung auf die Keimreduktion und die befördernde Beeinflussung der Heilung. Diese Art der Beeinflussung von Stoffwechselprozessen und physiologischen Zuständen wird üblicherweise mit der Laserbestrahlung mit geringer mittlerer Leistung im Milliwattbereich assoziiert ("Low Level Laser Therapy"). Diese biostimulative Art der Wirkung steht jedoch nicht im Zentrum der vorliegenden Erfindung.

Insgesamt lässt sich die als Erfindung hier vorgestellte Vorrichtung und Methode als "kalte" Laser-Wundreinigung und -Keimreduktion charakterisieren.

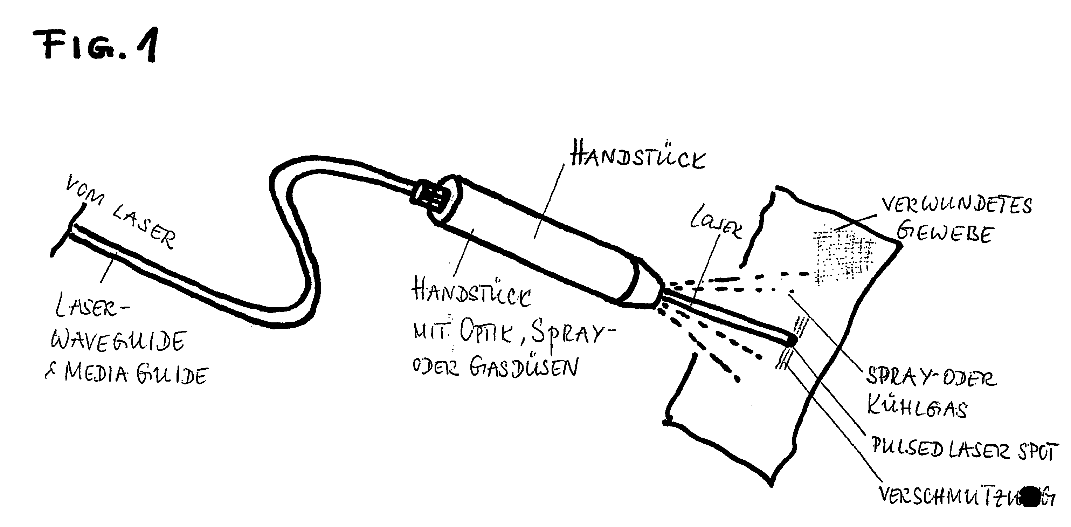

In Fig. 1 ist ein Handstück für eine Ausführungsform der erfindungsgemäßen Vorrichtung dargestellt. Bei dieser Ausführungsform wird ein gepulster Laserstrahl auf ein verwundetetes Gewebe gerichtet, wobei die Wunde verschmutzt ist. Die erfindungsgemäße Vorrichtung dient dabei der Reinigung der Wunde. Der Laserstrahl wird dabei von Strahlen mit einem Spülmittel, z.B. Desinfektionsmittel enthaltend umgeben. Düsen sorgen dabei für eine genaue Ausrichtung auf das Ziel. Im Handstück selbst ist eine Optik und die Düsen für das Spülmittel angeordnet. Das Laserlicht wird hier über eine Leitung zum Handstück geführt. Alternativ kann die Lasererzeugung auch im Handstück erfolgen, wenn ein geeigneter Laser verwendet wird.

## Patentansprüche

1. Vorrichtung zur Reinigung und Keimreduktion von offenen Wunden mit einem gütegeschalteten Festkörperlaser mit Neodym-dotiertem Lasermedium zur Erzeugung mindestens einer gepulsten Laserstrahlung, wobei der Laser wenigstens teilweise Strahlung im Wellenlängenbereich 600 - 750 nm erzeugt,
**dadurch gekennzeichnet, dass**
der Festkörperlaser eine Frequenzverdoppelung mit einer Grundwellenlänge bei 1330 ±100 nm und einer Verdoppelung zu 665 ±50 nm aufweist.

2. Vorrichtung nach mindestens einem der vorhergehenden Ansprüche, **dadurch gekennzeichnet, dass** Laserstrahlung der Grundwellenlänge und der zweiten Harmonischen synchron appliziert werden.

3. Vorrichtung nach Anspruch 2, **dadurch gekennzeichnet, dass** der Energieanteil der zweiten Harmonischen an der Gesamtenergie 15 bis 35% beträgt.

4. Vorrichtung nach mindestens einem der vorherigen Ansprüche, **dadurch gekennzeichnet, dass** der Festkörperlaser als Nd : YAG Laser ausgebildet ist.

5. Vorrichtung nach mindestens einem der vorhergehenden Ansprüche, **dadurch gekennzeichnet, dass** ein gütegesehalteter Laser mit faseroptischer Resonatorverlängerung und mit partieller oder vollständiger Frequenzverdoppelung mit Pulsdauern im Bereich 200 ns bis 2 *µ*s, bevorzugt 300 bis 600 ns, bei 50 bis 100 mJ Pulsenergie, verwendet wird.

6. Vorrichtung nach mindestens einem der vorhergehenden Ansprüche, **dadurch gekennzeichnet, dass** ein blitzlampengepumpter Laser verwendet wird.

7. Vorrichtung nach mindestens einem der vorhergehenden Ansprüche, **dadurch gekennzeichnet, dass** ein Dioden- bzw. Diodenlaser-gepumpter Festkörperlaser verwendet wird.

8. vorrichtung nach mindestens einem der vorhergehenden Ansprüche, **dadurch gekennzeichnet, dass** die Laserstrahlung mit einem Spiegelgelenkarm Faser appliziert wird.

9. Vorrichtung nach mindestens einem der vorhergehenden Ansprüche, **dadurch gekennzeichnet, dass** die Laserstrahlung mit einer optischen Faser appliziert wird.

10. Vorrichtung nach mindestens einem der vorhergehenden Ansprüche, **dadurch gekennzeichnet, dass** die Laserstrahlung mit einer optischen Faser mit optisch planer oder sphärisch ausgebildeter Endfläche appliziert wird.

11. Vorrichtung nach mindestens einem der Ansprüche 9 bis 10 **dadurch gekennzeichnet, dass** ein faseroptisches oder am Ende des Gelenkarmes angebrachtes Handstück zur Strahlungsapplikation verwendet wird.

12. Vorrichtung nach mindestens einem der vorhergehenden Ansprüche, **gekennzeichnet durch** ein Mittel, insbesondere einem Handstück, zur Zuführung von sterilem Spülmittel, insbesondere Spülgas, unter Druck und Düsen, die das Spülgas auf den wirkort der Laserstrahlung richten.

13. Vorrichtung nach mindestens einem der vorhergehenden Ansprüche, **dadurch gekennzeichnet, dass** das Mittel zur Zuführung von sterilem Spülmittel ein Mittel, insbesondere Düsen aufweist, mit dem das Spülmittel in vernebelter oder Tröpfchenform oder als Strahl auf den Wirkort des Laserstrahles richtbar ist.

14. Vorrichtung nach mindestens einem der vorhergehenden Ansprüche, **dadurch gekennzeichnet, dass** das Spülmittel oder das Spülgas im gekühltem Zustand zugeführt werden.

15. Vorrichtung nach mindestens einem der vorhergehenden Ansprüche, **dadurch gekennzeichnet, dass** dem Spülmittel oder dem Spülgas ein flüssiges Kältemittel zugesetzt ist, das beim Auftreffen verdampft und dadurch die Wunde kühlt.

16. Vorrichtung nach mindestens einem der vorhergehenden Ansprüche, **dadurch gekennzeichnet, dass** dem Spülmittel oder dem Spülgas Medikamente oder heilungsfördernde Stoffe zugesetzt sind, die beim Auftreffen der Wundoberfläche zugeführt werden und dort eindiffundieren oder auf andere weise resorbiert werden.

17. Vorrichtung nach mindestens einem der vorhergehenden Ansprüche, **dadurch gekennzeichnet, dass** dem Spülmittel oder dem Spülgas Stoffe zugesetzt sind, die keimreduzierend wirken und beim Auftreffen die Keimbelastung der Wundoberfläche senken.

## Claims

1. Apparatus for cleaning and germ reduction for open wounds using a Q-switched solid state laser with a neodymium-doped laser medium for producing at least one pulsed laser radiation, with the laser producing at least part of its radiation in the wavelength band from 600 to 750 nm,
**characterized in that**
the solid state laser has a frequency doubling effect, with a fundamental wavelength of 1330 ± 100 nm, which is doubled to 665 ± 50 nm.

2. Apparatus according to at least one of the preceding claims, **characterized in that** laser radiation at the fundamental wavelength and at the second harmonic are applied synchronously.

3. Apparatus according to Claim 2, **characterized in that** the energy component of the second harmonic is 15 to 35% of the total energy.

4. Apparatus according to at least one of the preceding claims, **characterized in that** the solid state laser is in the form of an Nd : YAG laser.

5. Apparatus according to at least one of the preceding claims, **characterized in that** a Q-switched laser is used with fibre-optic resonator lengthening and with partial or complete frequency doubling with pulse durations in the range from 200 ns to 2 µs, preferably 300 to 600 ns, with a pulse energy of 50 to 100 mJ.

6. Apparatus according to at least one of the preceding claims, **characterized in that** a flashlamp-pumped laser is used.

7. Apparatus according to at least one of the preceding claims, **characterized in that** a diode-pumped solid state laser or a diode laser-pumped solid state laser is used.

8. Apparatus according to at least one of the preceding claims, **characterized in that** the laser radiation is applied by means of a mirrored hinged arm fibre.

9. Apparatus according to at least one of the preceding claims, **characterized in that** the laser radiation is applied by means of an optical fibre.

10. Apparatus according to at least one of the preceding claims, **characterized in that** the laser radiation is applied by means of an optical fibre with an optically planar or spherical end surface.

11. Apparatus according to at least one of Claims 9 to 10, **characterized in that** a fibre-optic handpiece, or a handpiece which is fitted to the end of the hinged arm, is used for radiation application.

12. Apparatus according to at least one of the preceding claims, **characterized by** a means, in particular a handpiece, for supplying a sterile rinsing agent, in particular rinsing gas, under pressure, and nozzles which direct the rinsing gas onto the point at which the laser radiation is acting.

13. Apparatus according to at least one of the preceding claims, **characterized in that** the means for supplying sterile rinsing agent has a means, in particular nozzles, using which the rinsing agent can be directed onto the point at which the laser beam is acting in the form of a mist, droplets or a jet.

14. Apparatus according to at least one of the preceding claims, **characterized in that** the rinsing agent or the rinsing gas is supplied in a cooled state.

15. Apparatus according to at least one of the preceding claims, **characterized in that** a liquid cooling agent, which vaporizes on impact and thus cools the wound, is added to the rinsing agent or to the rinsing gas.

16. Apparatus according to at least one of the preceding claims, **characterized in that** medicaments or substances which promote healing are added to the rinsing agent or to the rinsing gas and are supplied on impact with the wound surface and where they diffuse in or are reabsorbed in some other way.

17. Apparatus according to at least one of the preceding claims, **characterized in that** substances which act to reduce germs and reduce the germ load on the wound surface on impact are added to the rinsing agent or to the rinsing gas.

## Revendications

1. Dispositif pour le nettoyage et la diminution de la quantité de germes de plaies ouvertes avec un laser solide à mode déclenché comportant un fluide laser dopé en néodyme pour la production d'au moins un rayonnement laser pulsé, le laser produisant au moins en partie un rayonnement d'une longueur d'onde de l'ordre de 600 à 750 nm,
**caractérisé en ce que**
le laser solide présente un doublement de la fréquence d'une longueur d'onde de base de 1330 ± 100 nm et ayant un doublement à 665 ± 50 nm.

2. Dispositif selon au moins l'une quelconque des revendications précédentes, **caractérisé en ce qu'**on applique de manière synchrone un rayonnement laser d'une longueur d'onde de base et un rayonnement laser de deuxième harmonique.

3. Dispositif selon la revendication 2, **caractérisé en ce que** la fraction énergétique du deuxième harmonique dans l'énergie totale est de 15 à 35 %.

4. Dispositif selon au moins une quelconque des revendications précédentes, **caractérisé en ce que** le laser solide est configuré comme un laser Nd:Yag.

5. Dispositif selon au moins l'une quelconque des revendications précédentes, **caractérisé en ce qu'**on utilise un laser à mode déclenché comportant un prolongement de résonateur à fibres optiques et un doublement partiel ou complet de la fréquence avec des impulsions d'une durée de l'ordre de 200 ns à 2 µs, de préférence de 300 à 600 ns, à une énergie d'impulsion de 50 à 100 mJ.

6. Dispositif selon au moins l'une quelconque des revendications précédentes, **caractérisé en ce qu'**on utilise un laser pompé par flash.

7. Dispositif selon au moins l'une quelconque des revendications précédentes, **caractérisé en ce qu'**on utilise un laser solide pompé par diodes respectivement par diodes laser.

8. Dispositif selon au moins l'une quelconque des revendications précédentes, **caractérisé en ce qu'**on applique le rayonnement laser avec un bras articulé à miroir.

9. Dispositif selon au moins l'une quelconque des revendications précédentes, **caractérisé en ce qu'**on applique le rayonnement laser avec une fibre optique.

10. Dispositif selon au moins l'une quelconque des revendications précédentes, **caractérisé en ce qu'**on applique le rayonnement laser avec une fibre optique ayant une face terminale optiquement plane ou sphérique.

11. Dispositif selon au moins l'une quelconque des revendications 9 à 10, **caractérisé en ce qu'**on utilise une pièce à main à fibre optique ou placée à l'extrémité du bras articulé pour appliquer le rayonnement.

12. Dispositif selon au moins l'une quelconque des revendications précédentes, **caractérisé par** un moyen, en particulier une pièce à main, pour l'acheminement d'un agent de rinçage stérile, en particulier un gaz de rinçage, sous pression et par des tuyères qui dirigent le gaz de rinçage sur le lieu d'action du rayon laser.

13. Dispositif selon au moins l'une quelconque des revendications précédentes, **caractérisé en ce que** le moyen d'acheminement de l'agent de rinçage stérile présente un moyen, en particulier des tuyères, avec lequel l'agent de rinçage peut être dirigé sur le lieu d'action du rayon laser sous forme nébulisée ou de gouttelettes ou sous forme de jet.

14. Dispositif selon au moins l'une quelconque des revendications précédentes, **caractérisé en ce que** l'agent de rinçage ou le gaz de rinçage est acheminé en l'état refroidi.

15. Dispositif selon au moins l'une quelconque des revendications précédentes, **caractérisé en ce qu'**on ajoute à l'agent de rinçage ou au gaz de rinçage un réfrigérant liquide qui se vaporise au moment de l'impact rafraîchissant ainsi la plaie.

16. Dispositif selon au moins l'une quelconque des revendications précédentes, **caractérisé en ce qu'**on ajoute à l'agent de rinçage ou au gaz de rinçage des médicaments ou des substances favorisant la guérison qui sont acheminées au moment de l'impact à la surface de la plaie et se diffusent à cet endroit ou sont résorbées d'une autre manière.

17. Dispositif selon au moins l'une quelconque des revendications précédentes, **caractérisé en ce qu'**on ajoute à l'agent de rinçage ou au gaz de rinçage des substances qui ont une action réduisant la quantité de germes et, au moment de l'impact, diminuent la sollicitation exercée par les germes à la surface de la plaie.
